# EUROPÄISCHE PATENTSCHRIFT

(11) **EP 2 452 656 B1**
(45) Veröffentlichungstag und Bekanntmachung des Hinweises auf die Patenterteilung: **30.04.2014**
(21) Anmeldenummer: 10014659.6
(22) Anmeldetag: 16.11.2010
(51) Int. Cl.: A61F 5/37

(54) **Fixierbandage zur Fixierung eines Patientes**
Fixing bandage for fixing a patient
Bandage fixe pour la fixation d'un patient

(43) Veröffentlichungstag der Anmeldung: 16.05.2012
(73) Patentinhaber: Wysozki, Roman, 22763 Hamburg (DE)
(72) Erfinder: Wysozki, Roman, 22763 Hamburg (DE)
(74) Vertreter: Hauck Patent- und Rechtsanwälte

(56) Entgegenhaltungen:
- DE-U1- 20 317 701
- DE-U1-202006 009 390
- DE-U1-202006 009 399
- JP-A- 2004 130 039

## Beschreibung

Die Erfindung betrifft eine Fixierbandage zur Fixierung eines Patienten auf einem Bett oder einem sonstigen Lager.

Eine zu diesem Zweck bekannte Fixierbandage weist einen flach auf das Bett aufzulegenden Bettgurt auf, der zur Befestigung an einem Bettgestell des Betts an jedem seiner beiden Enden eine Betthalterung aufweist. Die Betthalterungen werden jeweils schlaufenförmig um einen Holm des Bettgestells herumgeschlungen und mit einer geeigneten Verriegelung, beispielsweise einem Magnetschloss, geschlossen. Dadurch ist der Bettgurt an dem Bett befestigt. An dem Bettgurt ist ein Leibgurt befestigt, der ringförmig um die Taille des Patienten herumgelegt und mit einer weiteren Verriegelung geschlossen werden kann. Dadurch ist der Patient an der Taille auf dem Bett fixiert. Dies schützt den Patienten vor einem Herausfallen aus dem Bett, ohne seine Bewegungsfreiheit insbesondere an den Händen und Füßen unnötig einzuschränken. Eine derartige Fixierbandage ist in dem Gebrauchsmuster DE 203 17 701 U1 beschrieben.

Um eine bessere Fixierung des Patienten zu erzielen, ist aus dem Gebrauchsmuster DE 20 2006 009 390 U1 eine Fixierbandage mit einem zusätzlichen Fixiergurt bekannt, der an dem Leibgurt und seitlich an den Betthalterungen befestigt wird.

Die in dem Gebrauchsmuster DE 203 17 701 U1 beschriebene Fixierbandage hat zur zusätzlichen seitlichen Befestigung zwei an einem ersten Punkt fest mit dem Leibgurt verbundene Seitenbefestigungen, deren freie Enden an einem zweiten Punkt an den Betthalterungen verriegelt werden. Die an dem Leibgurt befestigten Enden der Seitenbefestigungen weisen jeweils einen Ring auf, durch den ein auf den Leibgurt aufgenähter und eine Schlaufe bildender Gurt hindurchgeführt ist. Diese Seitenbefestigungen können zuverlässig verhindern, dass der Patient sich auf die Seite dreht. Dies ist in vielen Fällen aus Sicherheitsgründen unbedingt erforderlich, um ein seitliches Herauslehnen oder Herausrutschen des Oberkörpers aus dem Bett zu verhindern, wobei es zu Verletzungen durch den Leibgurt kommen kann.

Aus der Druckschrift JP 2004 130 039 A ist eine Fixierbandage für einen Säugling bekannt geworden. Sie soll verhindern, dass sich der Säugling während des Wechselns seiner Windel umdreht. Die bekannte Fixierbandage weist einen Bettgurt auf, der um die Matratze herumgeführt ist. Ein weiterer Gurt ist über den Bauch des Säuglings geführt. Außerdem gibt es zwei seitlich verschiebliche Schnallen, durch die der Bettgurt und der weitere Gurt hindurchgeführt sind.

Davon ausgehend ist es die Aufgabe der Erfindung, eine Fixierbandage zur Verfügung zu stellen, die in der Praxis eine sicherere Fixierung eines Patienten ermöglicht.

Diese Aufgabe wird gelöst durch die Fixierbandage zur Fixierung eines Patienten auf einem Bett oder einem sonstigen Lager mit den Merkmalen des Anspruchs 1. Vorteilhafte Ausgestaltungen sind in den sich anschließenden Unteransprüchen angegeben.

Die Fixierbandage hat
- einen flach auf eine Liegefläche des Betts aufzulegenden Bettgurt, der zur Befestigung an einem Bettgestell des Betts an jedem seiner beiden Enden eine Betthalterung aufweist,
- einen ringförmig um einen Körperteil des Patienten zu schließenden Körperteilgurt, der an dem Bettgurt befestigt ist, und
- zwei Seitenbefestigungen zur zusätzlichen seitlichen Befestigung des Körperteilgurts, wobei ein erstes Ende jeder Seitenbefestigung an einem ersten Punkt an dem Körperteilgurt und ein zweites Ende jeder Seitenbefestigung an einem zweiten Punkt an dem Bettgurt oder der Betthalterung befestigt oder befestigbar ist, wobei
- einer Seitenbefestigung ein Seitenbefestigungshaltemittel zugeordnet ist, das zum Niederhalten der Seitenbefestigung zwischen dem ersten Punkt und dem zweiten Punkt ausgebildet ist.

Die erfindungsgemäße Fixierbandage dient zur Fixierung eines Patienten auf einem Bett oder einem sonstigen Lager. Mit Fixierung ist einerseits eine Ruhigstellung verwirrter, unkooperativer oder nicht zurechnungsfähiger Patienten gemeint. Einbezogen ist darüber hinaus eine Lagerung von Patienten in einer bevorzugten Haltung aus anderen Gründen, beispielsweise zur Decubitus-Prophylaxe. Das sonstige Lager kann beispielsweise eine Trage oder eine Liege sein, beispielsweise für einen Krankentransport. Soweit in den Anspruchsmerkmalen auf Elemente des Betts wie das Bettgestell oder einen Holm des Bettgestells Bezug genommen wird, geschieht dies der Einfachheit halber stellvertretend für entsprechende Komponenten anderer Lager. Beispielsweise kann das Bettgestell im Falle einer Trage ein Rahmen der Trage sein und der Holm des Bettgestells ein seitlicher Träger dieses Rahmens.

Der Bettgurt wird flach auf eine Liegefläche des Betts, beispielsweise auf eine Matratze, aufgelegt. Die Betthalterungen können Gurte sein, deren eines Ende mit einem Ende des Bettgurts verbunden sind, insbesondere durch Vernähen, aber auch lösbar. Sie können auch einteilig mit dem Bettgurt ausgebildet sein, sodass ein einziger Gurt mit seinem mittleren Abschnitt den Bettgurt und mit seinen beiden sich daran anschließenden Abschnitten die beiden Betthalterungen bildet. Zur Befestigung an einem Bettgestell des Betts (oder, beispielsweise, an einem Träger eines Rahmens einer Trage) können die Betthalterungen insbesondere um einen Bettholm des Bettgestells herumgeführt und die so gebildete Schlaufe von einer geeigneten Verriegelung, beispielsweise einem Magnetschloss, das durch zwei Ösen in der Betthalterung hindurchgesteckt wird, geschlossen werden.

Der Körperteilgurt umschließt im Gebrauch einen Körperteil des Patienten, beispielsweise die Taille. Er kann relativ breit und gegebenenfalls gepolstert ausgeführt sein, um einen hohen Tragekomfort zu bieten. Er kann eine Einstellmöglichkeit aufweisen, beispielsweise in Form von in Umfangsrichtung beabstandeten Ösen, durch die eine Verriegelung hindurchgeführt werden kann. Dadurch kann der Körperteilgurt stets ausreichend fest angelegt werden, ohne zu unbequem zu sein.

Der Körperteilgurt ist an dem Bettgurt befestigt. Er kann beispielsweise unmittelbar mit dem Bettgurt vernäht sein, insbesondere entlang einer quer zur Längsrichtung der Gurte verlaufenden Mittellinie des Bettgurts und des Körperteilgurts. Die Verbindung kann sich auch über einen Längsabschnitt des Körperteilgurts und des Bettgurts erstrecken. Die Befestigung des Körperteilgurts an dem Bettgurt kann auch mit Hilfe von weiteren Gurten erfolgen, die sich über einen Längsabschnitt des Körperteilgurts und des Bettgurts erstrecken und im Wesentlichen an ihren Enden mit jeweils einem der beiden Gurte verbunden sind. Derartige Gurte können kreuzweise angeordnet sein, sodass der ringförmig geschlossene Körperteilgurt ein Stück weit auf dem Bettgurt abgerollt werden kann, um eine begrenzte Drehbewegung des Patienten zuzulassen.

Die Seitenbefestigungen können ebenfalls insbesondere von Gurten gebildet sein. Ebenfalls möglich ist eine Ausführung, in der die beiden Seitenbefestigungen von einem einzigen Gurt gebildet werden, dessen mittlerer Abschnitt am Körperteilgurt fixiert wird. Die Fixierung kann beispielsweise mit einem Magnetschloss erfolgen. Bei der Verwendung eines einzigen Gurts für beide Seitenbefestigungen können dann die beiden ersten Punkte zusammenfallen. Die beiden Seitenbefestigungen können auch von zwei getrennten Gurten gebildet werden, deren beide Enden an den jeweiligen ersten und zweiten Punkten befestigt oder befestigbar sind. Am ersten Punkt und am zweiten Punkt befestigt oder befestigbar bedeutet, dass die Seitenbefestigung an den beiden Punkten Zugkräfte vom Körperteilgurt bzw. vom Bettgurt oder einer Betthalterung aufnehmen kann. Insbesondere können die beiden Seitenbefestigungen an ihren ersten Enden jeweils fest an ersten Punkten an dem Körperteilgurt befestigt sein, beispielsweise durch Vernähen oder mit Hilfe eines Verbindungsrings, durch die Seitenbefestigung eine Schlaufe bildend hindurchgeführt ist, und durch den außerdem ein beispielsweise auf den Körperteilgurt aufgenähter schmalerer Gurt hindurchgeführt ist. Natürlich kann ein solcher Verbindungsring auch auf andere Weise an dem Körperteilgurt oder der Seitenbefestigung befestigt sein.

Die Befestigung der Seitenbefestigungen an den zweiten Punkten kann an dem Bettgurt oder an der Betthalterung erfolgen. Insbesondere kann diese Befestigung von einer lösbaren Verriegelung, insbesondere wieder mit einem Magnetschloss, gebildet sein. Die Seitenbefestigungen können, ebenso wie die Betthalterungen und der Körperteilgurt, mehrere Ösen aufweisen oder sonstige Verbindungsmittel, die ein Anbringen der genannten Befestigungen an unterschiedlichen Positionen ermöglichen. Die zweiten Punkte befinden sich im Gebrauch der Fixierbandage in einem seitlichen Abstand von den ersten Punkten, sodass die Seitenbefestigungen eine seitliche Bewegung des Körperteilgurts und somit eine Drehbewegung des Patienten unterbinden.

Bei der geschilderten Anordnung bildet sich zwischen den Seitenbefestigungen und der Liegefläche bzw. dem Bettgurt/den Betthalterungen eine annähernd dreieckige Öffnung aus. Eine obere Seite der die Öffnung umschreibenden Dreiecke wird von jeweils einer Seitenbefestigung gebildet, eine untere Seite von einem Abschnitt des Bettgurts und/oder der Betthalterung. Die untere Seite kann im Wesentlichen auf der Liegefläche des Betts aufliegen. Die Erfindung beruht auf der Erkenntnis, dass diese dreieckige Öffnung eine Gefährdung für einen fixierten Patienten darstellt, weil der Patient mit einem nicht vom Körperteilgurt fixierten anderen Körperteil unter Umständen in diese Öffnung hineingeraten kann. Diese Gefahr besteht insbesondere, wenn der Körperteilgurt ein um die Taille des Patienten geschlungener Leibgurt ist. In diesem Fall kann der Patient mit dem Kopf oder Hals in die dreieckige Öffnung gelangen. In Einzelfällen kann sich der Patient dabei strangulieren. Auch wenn mit dem Körperteilgurt ein anderes Körperteil fixiert wird, stellen die dreieckigen

Öffnungen eine mögliche Ursache für Verletzungen oder sonstige Beeinträchtigungen dar.

Die Erfindung setzt dieser Gefahr mindestens ein Seitenbefestigungshaltemittel entgegen, das einer der Seitenbefestigungen zugeordnet und zum Niederhalten der Seitenbefestigung zwischen dem ersten Punkt und dem zweiten Punkt ausgebildet ist. Niederhalten bedeutet, dass die Seitenbefestigung zwischen dem ersten und dem zweiten Punkt von dem Seitenbefestigungshaltemittel nach unten gezogen oder unten gehalten wird. Die Seitenbefestigung verläuft daher zwischen den beiden Punkten nicht mehr geradlinig, sondern zumindest teilweise unterhalb einer geraden Linie zwischen den beiden Punkten. Dadurch wird das geschilderte Dreieck, das als Ursache der möglichen Gefährdung erkannt wurde, verkleinert und/oder zumindest teilweise geschlossen. Es ist dann nicht mehr ohne Weiteres möglich, einen anderen Körperteil, insbesondere den Kopf, in oder durch die Öffnung zu stecken.

Ein weiterer Vorteil der Erfindung ist, dass eine bestehende Fixierbandage einfach gemäß der Erfmdung nachgerüstet werden kann. Hierzu kann eine bestehende Fixierbandage, die noch keine Seitenbefestigungen aufweist, mit erfindungsgemäßen Seitenbefestigungen und Seitenbefestigungshaltemitteln versehen werden. Sofern eine bestehende Fixierbandage bereits herkömmliche Seitebefestigungen hat, können diese mit erfindungsgemäß ausgebildeten Seitenbefestigungshaltemitteln nachgerüstet werden.

In einer Ausgestaltung ist das Seitenbefestigungshaltemittel unterhalb des ersten Punkts an dem Körperteilgurt befestigt, sodass es die Seitenbefestigung zwischen dem ersten Punkt und dem zweiten Punkt an den Körperteilgurt heranziehen kann. Durch die Befestigung des Seitenbefestigungshaltemittels unterhalb des ersten Punkts kann es die Seitenbefestigung schräg nach unten an den Körperteilgurt heranziehen.

In einer Ausgestaltung ist das Seitenbefestigungshaltemittel an dem Bettgurt und/oder an der Betthalterung befestigt, sodass es die Seitenbefestigung zwischen dem ersten Punkt und dem zweiten Punkt an den Bettgurt und/oder die Betthalterung heranziehen kann. In diesem Fall kann es die Seitenbefestigung insbesondere unmittelbar nach unten, an den Bettgurt und/oder die Betthalterung heran, d.h. gleichsam in Richtung zu der Liegefläche hin, ziehen.

In einer Ausgestaltung ist das Seitenbefestigungshaltemittel in ungefähr gleich großem Abstand von dem ersten Punkt und dem zweiten Punkt angeordnet. Dadurch wird das Dreieck etwa in der Mitte von dem Seitenbefestigungshaltemittel zusammengezogen, sodass im Allgemeinen keine der beiden verbleibenden Hälften noch eine Gefahr darstellt.

In einer Ausgestaltung ist das Seitenbefestigungshaltemittel näher an dem ersten Punkt als an dem zweiten Punkt angeordnet. In diesem Fall wirkt das Seitenbefestigungshaltemittel anders als bei einer mittigen Anordnung zwischen dem ersten und dem zweiten Punkt auf einen Punkt der Seitenbefestigung ein, der sich - bei geradlinigem Verlauf der Seitenbefestigung, wie er sich ohne das Seitenbefestigungshaltemittel ergeben würde — in relativ großem Abstand von der Liegefläche befindet. Dadurch wird die erläuterte Verletzungsgefahr in vielen Fällen besonders wirksam verringert.

In einer Ausgestaltung ist das Seitenbefestigungshaltemittel so angeordnet, dass die Seitenbefestigung zwischen dem ersten Punkt und dem Seitenbefestigungshaltemittel im Gebrauch der Fixierbandage in einem Winkel im Bereich von 30° bis 60° zur Liegefläche verläuft. Bevorzugt kann der Winkel ungefähr 45° betragen. In diesem Fall ist die Fläche des Dreiecks zwischen dem Körperteilgurt und dem Seitenbefestigungshaltemittel besonders klein, ohne dass die seitliche Fixierungswirkung der Seitenbefestigung unnötig eingeschränkt wird.

In einer Ausgestaltung sind einer Seitenbefestigung mehrere Seitenbefestigungshaltemittel zugeordnet. Weiter bevorzugt sind die mehreren Seitenbefestigungshaltemittel in gleichmäßigem Abstand voneinander angeordnet. Beide Ausgestaltungen verringern die Gefahr weiter, dass es zu Verletzungen durch die verbleibenden Öffnungen zwischen den unterschiedlichen Gurten kommt.

In einer Ausgestaltung sind einer Seitenbefestigung mindestens ein Seitenbefestigungshaltemittel, das am Körperteilgurt befestigt ist, und mindestens ein Seitenbefestigungshaltemittel, das am Bettgurt und/oder an der Betthalterung befestigt ist, zugeordnet. Bevorzugt kann jeweils genau ein Seitenbefestigungshaltemittel eingesetzt werden, sodass die Seitenbefestigung an zwei Punkten zwischen dem ersten Punkt und dem zweiten Punkt nach unten bzw. an den Körperteilgurt herangezogen wird. Dies stellt eine nach allen praktischen Erfahrungen ausreichende Vermeidung der genannten Verletzungsgefahr dar, ohne dass die Anordnung zu aufwendig und die Fixierbandage damit unnötig schwer anzulegen ist.

In einer Ausgestaltung weist ein Seitenbefestigungshaltemittel einen Ring oder eine Schlaufe auf, durch die die Seitenbefestigung hindurchführbar ist. In beiden Fällen kann die Seitenbefestigung in dem Seitenbefestigungshaltemittel frei verschieblich angeordnet sein. Dies vereinfacht das Anlegen der Fixierbandage, weil beim Fixieren der Seitenbefestigung, beispielsweise an ihrem zweiten Punkt, das Seitenbefestigungshaltemittel automatisch an eine für die Niederhaltefunktion günstige Position der Seitenbefestigung rutscht. Der Ring kann beispielsweise ein Rechteckring sein, insbesondere aus Metall. Die Schlaufe kann beispielsweise aus einem Gurtmaterial bestehen, das je nach Befestigungsort des Seitenbefestigungshaltemittels beispielsweise am Körperteilgurt, an der Betthalterung oder am Bettgurt vernäht sein kann.

In einer Ausgestaltung ist ein Seitenbefestigungshaltemittel ein Schlauch, durch den die Seitenbefestigung hindurchführbar ist. Der Schlauch kann beispielsweise aus einem textilen Material, etwa aus einem Gurtmaterial, bestehen. Durch das Hindurchführen durch einen solchen Schlauch ist die Seitenbefestigung über einen größeren Längsabschnitt geführt. Die frei verlaufenden Abschnitte der Seitenbefestigung sind dadurch verkürzt und die verbleibenden Öffnungen entsprechend verkleinert. Dabei kann das Hindurchführen der Seitenbefestigung durch einen Schlauch einfacher sein als durch mehrere, in einem Abstand voneinander angeordnete Haltemittel, etwa mehrere Ringe.

In einer Ausgestaltung ist der Schlauch in Längsrichtung mit dem Körperteilgurt oder dem Bettgurt und/oder einer der Betthalterungen vernäht. Dadurch ist der Schlauch sicher befestigt und das Hindurchführen der Seitenbefestigung wird vereinfacht.

In einer Ausgestaltung ist der Körperteilgurt ein Leibgurt, ein Brustgurt, ein Schultergurt, ein Oberschenkelgurt, ein Handgelenkgurt, ein Fußgelenkgurt oder ein Kopfgurt zum Umschließen des entsprechenden Körperteils. Zusätzliche Seitenbefestigungen haben sich insbesondere für Leibgurte bewährt. Sie sind jedoch auch für die anderen genannten Gurte sinnvoll, um eine optimale Fixierung zu erreichen. Die Erfindung ist für die genannten Gurtarten gleichermaßen geeignet.

In einer Ausgestaltung ist der Körperteilgurt ein Leibgurt und die Fixierbandage weist einen Schrittgurt, eine Oberschenkelhalterung, eine Schulterhalterung und/oder Handmanschetten oder Schlaufen zur Befestigung von Handhalterungen auf. Durch die zusätzlichen Elemente wird ein Herausrutschen des Patienten aus dem Leibgurt nach oben oder unten verhindert bzw. durch die Handmanschetten oder Schlaufen, die insbesondere an dem Bettgurt oder an den Befestigungsgurten angeordnet und befestigt sein können, wird eine besonders einfache zusätzliche Fixierung der Hände bzw. Arme des Patienten ermöglicht.

Die Erfindung wird nachfolgend anhand von in Figuren dargestellten Ausführungsbeispielen näher erläutert. Es zeigen:
- Fig. 1: eine erfindungsgemäße Fixierbandage im angelegten Zustand in einer vereinfachten Querschnittsdarstellung,
- Fig. 2: eine Alternative eines in Figur 1 dargestellten Details,
- Fig. 3: eine erfindungsgemäße Fixierbandage im angelegten Zustand in einer vereinfachten Querschnittsdarstellung, wobei die Figur nur einen Ausschnitt der gesamten Anordnung zeigt,
- Fig. 4: eine nicht erfindungsgemäß ausgestaltete Fixierbandage mit einer herkömmlichen Seitenbefestigung in einer der Figur 4 entsprechenden Darstellung.
Es werden in allen Figuren für sich entsprechende Teile die gleichen Bezugszeichen verwendet.

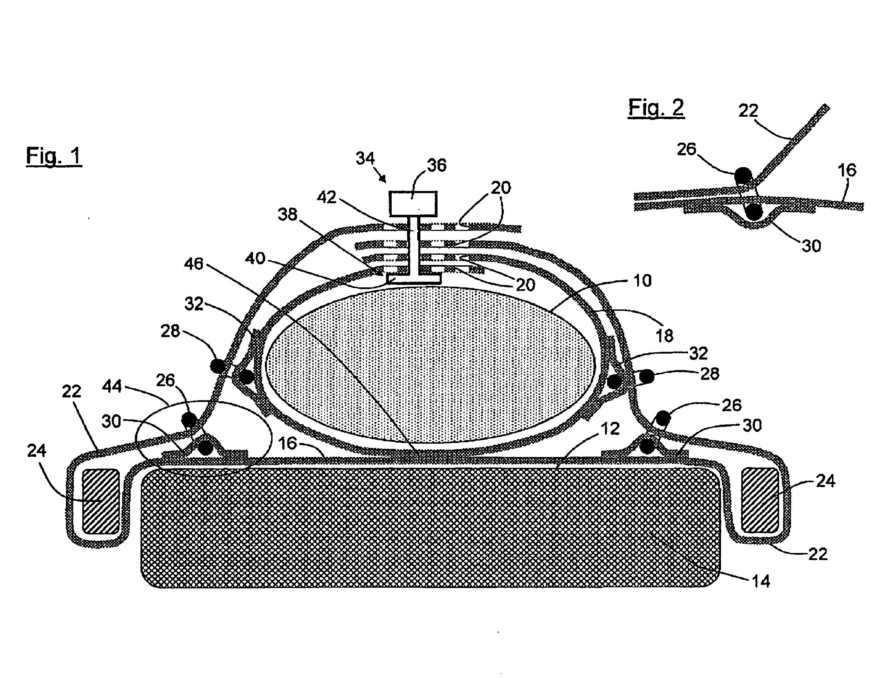

Fig. 1 zeigt die Taille 10 eines Patienten, der auf einer Liegefläche 12 einer Matratze 14 eines Betts liegt. Der Patient ist mit einer erfindungsgemäßen Fixierbandage fixiert, die einen auf der Liegefläche 12 aufliegenden Bettgurt 16 aufweist, der wie die übrigen Gurte auch aus einem textilen Gurtmaterial besteht. Der Bettgurt 16 erstreckt sich annähernd über die gesamte Breite der Liegefläche 12 und weist zwei Enden auf, die sich nahe den Kanten der Matratze 14 befinden.

In der Mitte des Bettgurts 16 ist bei 46 ein Leibgurt 18 befestigt, der die Taille 10 des Patienten ringförmig umschließt. Der Leibgurt 18 ist ein Körperteilgurt für den Körperteil "Taille" des Patienten. An beiden Enden des Leibgurts 18 sind jeweils mehrere Ösen 20 angeordnet, wobei im ringförmig geschlossenen Zustand des Leibgurts 18 mindestens zwei der Ösen 20 übereinander liegen.

Die Fixierbandage ist mit einem einzigen Magnetschloss 34 geschlossen. Das Magnetschloss 34 weist einen Schließknopf 36 und einen Sockel 38 mit einem Teller 40 und einem Schaft 42 auf. Der Teller 40 befindet sich unterhalb des unteren Endes des Leibgurts 18 und der Schaft 42 ist durch eine Öse 20 jeder der vier Gurtlagen hindurchgeführt. Oberhalb der obersten Gurtlage, die im Beispiel von dem in der Figur links angeordneten Befestigungsgurt 22 gebildet wird, ist der Schließknopf 36 aufgesteckt.

An jedes Ende des Bettgurts 16 schließt sich jeweils ein Befestigungsgurt 22 an, der ausgehend von dem jeweiligen Ende des Bettgurts 16 um einen Holm 24 des Bettgestells herumgeführt ist und von dort zu dem Leibgurt 18 zurückgeführt ist. Genauer erstreckt sich der Befestigungsgurt 22 von dem Holm 24 ausgehend durch zwei Seitenbefestigungshaltemittel in Form rechteckiger Ringe 26, 28 aus Metall hindurch und von dort weiter parallel zum Leibgurt 18 bis zu einer Oberseite des Leibgurts 18, wo die beiden Enden des Leibgurts 18 mit den Ösen 20 übereinanderliegen. Auch das freie Ende jedes Befestigungsgurts 22 weist Ösen 20 auf, von denen jeweils mindestens eine oberhalb einer Öse 20 des Leibgurts angeordnet ist.

Der Abschnitt des Befestigungsgurts 22 zwischen dem Holm 24 des Bettgestells und dem freien Ende, das von dem Magnetschloss 34 fixiert ist, ist eine Seitenbefestigung. Der erste Punkt dieser Seitenbefestigung ist mit dem Magnetschloss 34 am Leibgurt 18 befestigt. Der zweite Punkt der Seitenbefestigung ist im Bereich des Holms 24 des Bettgestells an einer Betthalterung befestigt, wo er in einen weiteren Abschnitt des Gurts übergeht, der diese Betthalterung bildet.

Die Seitenbefestigung wird niedergehalten durch zwei Seitenbefestigungshaltemittel. Das erste Seitenbefestigungshaltemittel besteht aus dem Ring 26 und einem Stück Gurtmaterial 30, mit dem der Ring 26 an der Oberseite des Bettgurts 16 befestigt ist, und zwar in einem im Gebrauch seitlich zwischen dem Leibgurt 18 und dem Holm 24 des Bettgestells befindlichen Abschnitt. Der Ring 26 zieht die Seitenbefestigung nach unten, an den Bettgurt 16 heran.

Die Ringe 28 sind Teil weiterer Seitenbefestigungshaltemittel, die jeweils an einer Seite des Leibgurts 18 befestigt sind, ebenfalls mit einem Stück Gurtmaterial 32. Die Seitenbefestigungen sind auch durch die Ringe 28 hindurchgeführt und werden durch diese schräg nach unten gezogen, an den Leibgurt 18 heran.

Figur 2 zeigt eine Alternative für den in Figur 1 mit 44 bezeichneten Ausschnitt, der die Seitenbefestigung mit dem Ring 26 enthält. Der Ring 26 ist ebenfalls an dem Bettgurt 16 befestigt, und die Seitenbefestigung in Form des Befestigungsgurts 22 ist durch den Ring 26 hindurchgeführt. Im Unterschied zur Figur 1 verläuft bei der in Figur 2 dargestellten Alternative auch der Bettgurt 16 selbst durch den Ring 26 hindurch, und ein aufgenähtes Stück Gurtmaterial 30 befindet sich an der Unterseite des Bettgurts 16. Es dient der Fixierung des Rings 26 an der gewünschten Position des Bettgurts 16.

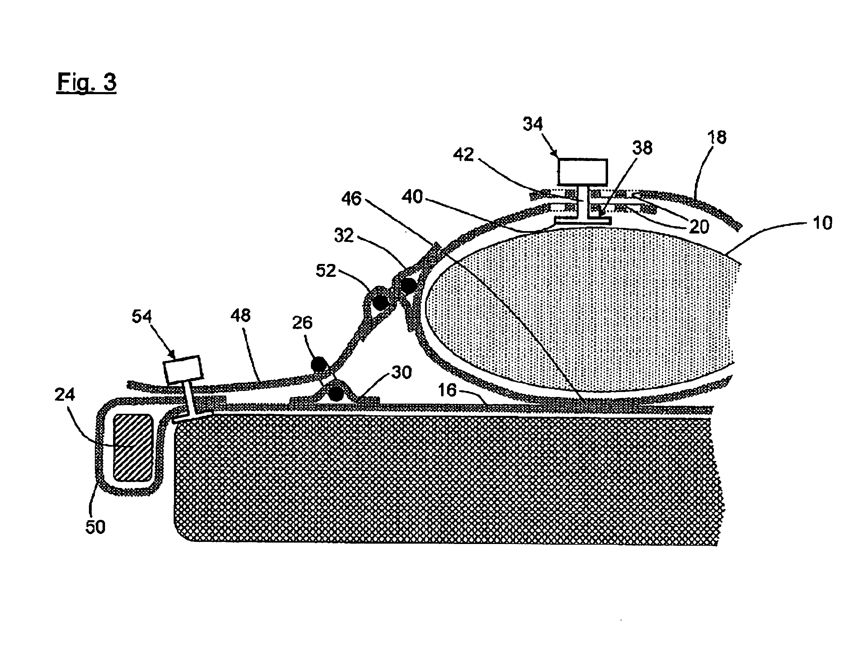

Figur 3 zeigt eine andere erfindungsgemäße Fixierbandage in einer der Figur 1 vergleichbaren vereinfachten Querschnittsdarstellung. Nur der auf einer Seite der Taille 10 des Patienten befindliche Teil der Anordnung ist gezeigt. Die Fixierbandage der Figur 3 weist ebenfalls einen Bettgurt 16 auf, der auf einer Liegefläche aufliegt und an der, wie in Verbindung mit Figur 1 bereits erläutert, ein Leibgurt 18 bei 46 befestigt ist. Das Magnetschloss 34 am Leibgurt 18 dient hier ausschließlich zum Schließen des Leibgurts 18 zu schließen. Daher ist der Schaft 42 des Sockels 38 kürzer ausgebildet als in Figur 1, nämlich angepasst an die beiden zu verbindenden Lagen des Leibgurts 18.

Der Bettgurt 16 weist an seinen beiden Enden jeweils eine Betthalterung 50 auf, die um einen Holm 24 des Bettgestells herumgeführt und mit einem weiteren Magnetschloss 54 schlaufenförmig geschlossen ist. Dadurch ist der Bettgurt 16 an beiden Enden mit jeweils einer Betthalterung 50 an dem Bettgestell fixiert.

Die beiden Seitenbefestigungen 48 werden jeweils von einem gesonderten Gurt gebildet. Dieser ist an einem ersten Punkt mit einem Ring 52, der rechteckig ist und aus Metall besteht, an dem Leibgurt 18 befestigt. Hierzu ist ein Ende der Seitenbefestigung 48 durch den Ring 52 hindurchgeführt und zu einer Schlaufe vernäht. Ebenfalls durch den Ring 52 hindurchgeführt ist ein Stück Gurtmaterial 32, dessen beide Enden mit dem Leibgurt 18 vernäht sind. Dadurch ist das eine Ende der Seitenbefestigung 48 an dem ersten Punkt fest mit dem Leibgurt 18 verbunden.

Das freie Ende der Seitenbefestigung 48 weist mehrere nicht im Einzelnen dargestellte Ösen auf, die in Längsrichtung der Seitenbefestigung 48 voneinander beabstandet sind. Durch zwei der Ösen ist der Schaft 42 des zum Schließen der Betthalterung 50 vorgesehenen Magnetschlosses 54 hindurchgeführt, sodass die Seitenbefestigung 48 an einem zweiten Punkt an der Betthalterung 50 befestigt ist. Zwischen dem ersten und dem zweiten Punkt ist ein Seitenbefestigungshaltemittel mit einem Ring 26 vorhanden, die die Seitenbefestigung 48 niederhält. Hierzu ist die Seitenbefestigung 48 durch den Ring 26 hindurchgeführt und wird von dem Ring 26 an den Bettgurt 16 herangezogen. Ebenfalls durch den Ring 26 hindurchgeführt ist ein Stück Gurtmaterial 30, dessen beide Enden mit dem Bettgurt 16 vernäht sind. Wie in der Figur 3 ersichtlich, sind auf beiden Seiten des Seitenbefestigungshaltemittels in Form des Rings 26 relativ kleine Öffnungen zwischen der Seitenbefestigung 48 und dem Bettgurt 16 vorhanden. Es besteht keine Gefahr, dass der Patient mit einem weiteren Körperteil in eine dieser Öffnungen hineingerät und sich dabei möglicherweise verletzt.

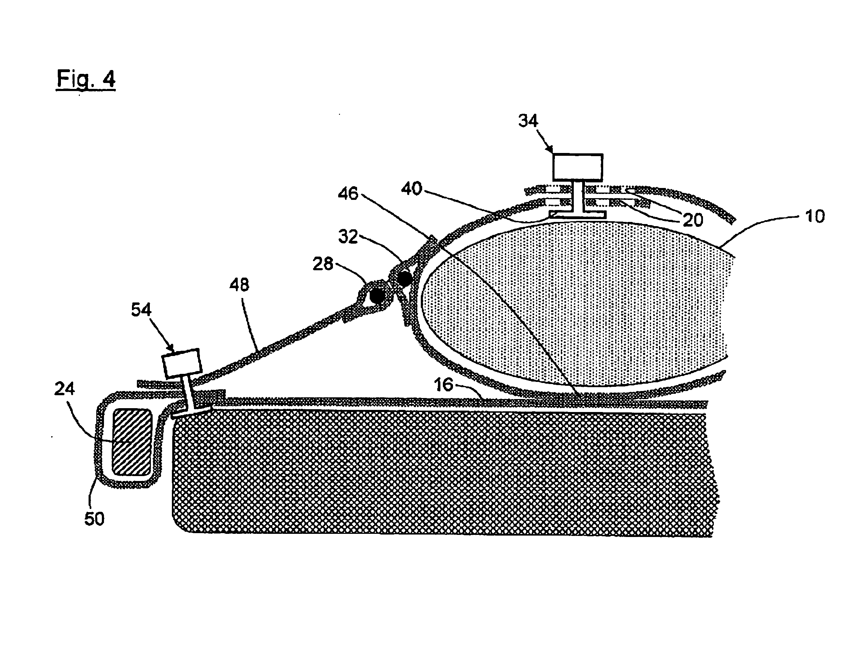

Figur 4 zeigt zum Vergleich eine nicht gemäß der Erfmdung ausgestaltete Fixierbandage. Bis auf das in Figur 3 vorhandene Seitenbefestigungshaltemittel in Form des Rings 26 ist diese Fixierbandage wie bereits erläutert aufgebaut. Gut erkennbar ist, dass die Seitenbefestigung 48 zwischen dem ersten Punkt am Ring 28 und dem zweiten Punkt an dem Magnetschloss 54, mit dem gleichzeitig die Betthalterung 50 geschlossen ist, straff gespannt ist. Unterhalb der Seitenbefestigung 48 befindet sich eine relativ große, dreieckige Öffnung, in die der Patient unter Umständen mit dem Kopf oder Hals hineingeraten kann.

### Liste der verwendeten Bezugszeichen:

- 10: Taille
- 12: Liegefläche
- 14: Matratze
- 16: Bettgurt
- 18: Leibgurt
- 20: Öse
- 22: Befestigungsgurt
- 24: Holm
- 26: Ring (am Bettgurt)
- 28: Ring (am Leibgurt)
- 30: Gurtmaterial zur Befestigung der Ringe 26 am Bettgurt
- 32: Gurtmaterial zur Befestigung der Ringe 28 am Leibgurt
- 34: Magnetschloss
- 36: Schließknopf
- 38: Sockel
- 40: Teller
- 42: Schaft
- 44: Ausschnitt aus Figur 1
- 46: Befestigungsbereich Bettgurt/Leibgurt
- 48: Seitenbefestigung
- 50: Betthalterung
- 52: Ring
- 54: weiteres Magnetschloss

## Patentansprüche

1. Fixierbandage zur Fixierung eines Patienten auf einem Bett oder einem sonstigen Lager mit
• einem flach auf eine Liegefläche (12) des Betts aufzulegenden Bettgurt (16), der zur Befestigung an einem Bettgestell des Betts an jedem seiner beiden Enden eine Betfhalterung (50) aufweist,
• einem ringförmig um einen Körperteil des Patienten zu schließenden Kölperteilgurt, der an dem Bettgurt (16) befestigt ist, und
• zwei Seitenbefestigungen (48) zur zusätzlichen seitlichen Befestigung des Körperteilgurts, wobei ein erstes Ende jeder Seitenbefestigung an einem ersten Punkt an dem Körperteilgurt und ein zweites Ende jeder Seitenbefestigung an einem zweiten Punkt an dem Bettgurt (16) oder der Betthalterung (50) befestigt oder befestigbar ist, **dadurch gekennzeichnet, dass**
• mindestens einer der Seitenbefestigungen (48) ein Seitenbefestigungshaltemittel zugeordnet ist, das zum Niederhalten der Seitenbefestigung (48) zwischen dem ersten Punkt und dem zweiten Punkt ausgebildet ist.

2. Fixierbandage nach Anspruch 1, **dadurch gekennzeichnet, dass** das Seitenbefestigungshaltemittel unterhalb des ersten Punkts an dem Körperteilgurt befestigt ist, so dass es die Seitenbefestigung (48) zwischen dem ersten Punkt und dem zweiten Punkt an den Körperteilgurt heranziehen kann.

3. Fixierbandage nach Anspruch 1, **dadurch gekennzeichnet, dass** das Seitenbefestigungshaltemittel an dem Bettgurt (16) und/oder an der Betthalterung (50) befestigt ist, so dass es die Seitenbefestigung (48) zwischen dem ersten Punkt und dem zweiten Punkt an den Bettgurt (16) und/oder die Betthalterung (50) heranziehen kann.

4. Fixierbandage nach einem der Ansprüche 1 bis 3, dadurch gekenntzeichnet, dass das Seitenbefestigungshaltemittel in ungefähr gleichgroßem Abstand von dem ersten Punkt und dem zweiten Punkt angeordnet ist.

5. Fixierbandage nach einem der Ansprüche 1 bis 4, **dadurch gekennzeichnet, dass** das Seitenbefestigungshaltemittel näher an dem ersten Punkt als an dem zweiten Punkt angeordnet ist.

6. Fixierbandage nach einem der Ansprüche 1 bis 5, **dadurch gekennzeichnet, dass** das Seitenbefestigungshaltemittel (26) so angeordnet ist, dass die Seitenbefestigung zwischen dem ersten Punkt und dem Seitenbefestigungshaltemittel im Gebrauch der Fixierbandage in einem Winkel im Bereich von 30° bis 60° zur Liegefläche verlauft.

7. Fixierbandage nach einem der Ansprüche 1 bis 6. **dadurch gekennzeichnet, dass** einer Seitenbefestigung (48) mehrere Seitenbefestigungshaltermittel zugeordnet sind.

8. Fixierbandage nach Anspruch 7, **dadurch gekennzeichnet, dass** die mehreren Seitenbefestigungsmittel in einem gleichmäßigen Abstand voneinander angeordnet sind.

9. Fixierbandage nach Anspruch 7 oder 8, **dadurch gekennzeichnet, dass** einer Seitenbefestigung (48) mindestens ein Seitenbefestigungshaltemittel, das am Körperteilgurt befestigt ist, und mindestens ein Seitenbefestigungshaltemittel, das am Bettgurt (16) und/oder an der Betthalterung (50) befestigt ist, zugeordnet sind.

10. Fixierbandage nach einem der Ansprüche 1 bis 9, **dadurch gekennzeichnet, dass** ein Seitenbefestigungshaltemittel einen Ring (26, 28) oder eine Schlaufe aufweist, durch die die Seitenbefestigung (48) hindurchführbar ist.

11. Fixierbandage nach einem der Ansprürhe 1 bis 10, **dadurch gekennzeichnet, dass** ein Seitenbe-festigungshalmittel einen Schlauch aufweist, durch den die Seitenbefestigung (48) hindurchführbar ist.

12. Fixierbandage nach Anspruch 11, **dadurch gekennzeichnet, dass** der Schlauch in Längsrichtung mit dem Körperteilgurt oder dem Hettgurt (16) und/oder einer der Betthalterungen (50) vernäht ist.

13. Fixierbandage nach einem der Ansprüche 1 bis 12, **dadurch gekennzeichnet, dass** der Körperteilgurt ein Leibgurt (18), ein Brustgurt, ein Schultergurt, ein Oberschenkelgurt, ein Handgelenkgurt, ein Fußgelenkgurt oder ein Kopfgurt zum Umschließen des entsprechenden Körperteils ist.

14. Fixierbandage nach einem der Ansprüche 1 bis 13, **dadurch gekennzeichnet, dass** der Körperteilgurt ein Leibgurt (18) ist und die Fixierbandage einen Schrittgurt, eine Oberschenkelhalterung, eine Schulterhalterung und/oder Handmanschetten oder Schlaufen zur Befestigung von Handhalterungen aufweist.

## Claims

1. A fixing bandage for fixing a patient on a bed or another repository, having
• a bed belt (16) to be laid flat on a lying surface (12) of the bed, that for fastening to a bed frame of the bed has a bed holder (50) on each of the ends thereof,
• an annular body part belt to be locked about a body part of the patient that is fastened to the bed belt (16), and
• two lateral fastenings (48) for additional lateral fastening of the body part belt, wherein a first end of each lateral fastening is or can be fastened to a first point on a body part belt, and a second end of each lateral fastening is or can be fastened to a second point on the bed belt (16) or the bed holder (50), **characterized in that**
• a lateral fastening holding means that is designed to hold down the lateral fastening (48) between the first point and the second point is assigned to at least one of the lateral fastenings (48).

2. The fixing bandage according to claim 1, **characterized in that** the lateral fastening holding means is fastened below the first point to the body part belt so that it can pull in the lateral fastening (48) between the first point and the second point on the body part belt.

3. The fixing bandage according to claim 1, **characterized in that** the lateral fastening holding means is fastened to the bed belt (16) and/or to be bed holder (50) so that it can pull in the lateral fastening (48) between the first point and the second point at the bed belt (16) and/or the bed holder (50).

4. The fixing bandage according to one of the claims 1 to 3, **characterized in that** the lateral fastening holding means is arranged at approximately equal distance from the first point and the second point.

5. The fixing bandage according to one of the claims 1 to 4, **characterized in that** the lateral fastening holding means is arranged closer to the first point than to the second point.

6. The fixing bandage according to one of the claims 1 to 5, **characterized in that** the lateral fastening holding means (26) is arranged so that the lateral fastening runs between the first point and the lateral fastening holding means during use of the fixing bandage at an angle in the range of 30° to 60° to the lying surface.

7. The fixing bandage according to one of the claims 1 to 6, **characterized in that** a plurality of lateral fastening holding means are assigned to a lateral fastening (48).

8. The fixing bandage according to claim 7, **characterized in that** the plurality of lateral fastening means are arranged at a uniform distance from each other.

9. The fixing bandage according to claim 7 or 8, **characterized in that** at least one lateral fastening holding means, which is fastened to the body part belt, and at least one lateral fastening holding means, which is fastened to the bed belt (16) and/or to the bed holder (50), are assigned to a lateral fastening (48).

10. The fixing bandage according to one of the claims 1 to 9, **characterized in that** a lateral fastening holding means has a ring (26, 28) or a loop through which the lateral fastening (48) can be passed.

11. The fixing bandage according to one of the claims 1 to 10, **characterized in that** a lateral fastening holding means has a hose through which the lateral fastening (48) can be passed.

12. The fixing bandage according to claim 11, **characterized in that** the hose in the longitudinal direction is sewn to the body part belt or the bed belt (16) and/or to one of the bed holders (50).

13. The fixing bandage according to one of the claims 1 to 12, **characterized in that** the body part belt is a waist belt (18), a chest belt, a shoulder belt, a thigh belt, a wrist belt, an ankle belt or a head belt for surrounding the corresponding body part.

14. The fixing bandage according to one of the claims 1 to 13, **characterized in that** the body part belt is a waist belt (18), and the fixing bandage has a crotch belt, a thigh holder, a shoulder holder and/or handcuffs or loops for fastening hand holders.

## Revendications

1. Bandage fixe pour la fixation d'un patient sur un lit ou sur un autre support, avec
• une sangle de lit (16) à placer à plat sur un plan de couchage (12) du lit et qui présente à chacune de ses deux extrémités une retenue de lit (50) pour la fixation sur un bâti de lit du lit,
• une sangle de partie corporelle à fermer de façon annulaire autour d'une partie corporelle du patient et qui est fixée sur la sangle de lit (16), et
• deux fixations latérales (48) destinées à la fixation latérale supplémentaire de la sangle de partie corporelle, une première extrémité de chaque fixation latérale étant fixée ou pouvant être fixée sur un premier point sur la sangle de partie corporelle, et une deuxième extrémité de chaque fixation latérale étant fixée ou pouvant être fixée sur un deuxième point sur la sangle de lit (16) ou sur la retenue de lit (50), **caractérisé en ce que**
• à au moins une des fixations latérales (48) est affecté un moyen de retenue de fixation latérale qui est constitué pour maintenir en bas la fixation latérale (48) entre le premier point et le deuxième point.

2. Bandage fixe selon la revendication 1, **caractérisé en ce que** le moyen de retenue de fixation latérale est fixé au-dessous du premier point sur la sangle de partie corporelle de telle sorte qu'il peut rapprocher par traction la fixation latérale (48) contre la sangle de partie corporelle entre le premier point et le deuxième point.

3. Bandage fixe selon la revendication 1, **caractérisé en ce que** le moyen de retenue de fixation latérale est fixé sur la sangle de lit (16) et/ou sur la retenue de lit (50) de telle sorte qu'il peut rapprocher par traction la fixation latérale (48) contre la sangle de lit (16) et/ou contre la retenue de lit (50) entre le premier point et le deuxième point.

4. Bandage fixe selon une des revendications 1 à 3, **caractérisé en ce que** le moyen de retenue de fixation latérale est disposé à une distance à peu près équivalente du premier point et du deuxième point.

5. Bandage fixe selon une des revendications 1 à 4, **caractérisé en ce que** le moyen de retenue de fixation latérale est disposé plus près du premier point que du deuxième point.

6. Bandage fixe selon une des revendications 1 à 5, **caractérisé en ce que** le moyen de retenue de fixation latérale (26) est disposé de telle sorte que la fixation latérale entre le premier point et le moyen de retenue de fixation latérale forme, pendant l'utilisation du bandage fixe, un angle dans la plage de 30° à 60° par rapport au plan de couchage.

7. Bandage fixe selon une des revendications 1 à 6, **caractérisé en ce que** plusieurs moyens de retenue de fixation latérale sont affectés à une fixation latérale (48).

8. Bandage fixe selon la revendication 7, **caractérisé en ce que** les plusieurs moyens de fixation latérale sont disposés à une distance régulière les uns des autres.

9. Bandage fixe selon la revendication 7 ou 8, **caractérisé en ce qu'**à une fixation latérale (48) sont affectés au moins un moyen de retenue de fixation latérale qui est fixé sur la sangle de partie corporelle et au moins un moyen de retenue de fixation latérale qui est fixé sur la sangle de lit (16) et/ou sur la retenue de lit (50).

10. Bandage fixe selon une des revendications 1 à 9, **caractérisé en ce qu'**un moyen de retenue de fixation latérale présente un anneau (26, 28) ou une boucle à travers lesquels peut être introduite la fixation latérale (48).

11. Bandage fixe selon une des revendications 1 à 10, **caractérisé en ce qu'**un moyen de retenue de fixation latérale présente un tuyau à travers lequel peut être introduite la fixation latérale (48).

12. Bandage fixe selon la revendication 11, **caractérisé en ce que** le tuyau est cousu dans la direction longitudinale avec la sangle de partie corporelle ou la sangle de lit (16) et/ou avec une des retenues de lit (50).

13. Bandage fixe selon une des revendications 1 à 12, **caractérisé en ce que** la sangle de partie corporelle est une sangle abdominale (18), une sangle de poitrine, une sangle d'épaule, une sangle de cuisse, une sangle de poignet, une sangle de cheville ou une sangle de tête destinée à entourer la partie corporelle correspondante.

14. Bandage fixe selon une des revendications 1 à 13, **caractérisé en ce que** la sangle de partie corporelle est une sangle abdominale (18), et **en ce que** le bandage fixe présente une sangle d'entrejambe, une retenue de cuisse, une retenue d'épaule et/ou des manchettes manuelles ou des boucles destinées à la fixation de retenues de main.
